# EUROPÄISCHE PATENTSCHRIFT

(11) **EP 0 578 236 B1**
(45) Veröffentlichungstag und Bekanntmachung des Hinweises auf die Patenterteilung: **26.11.1997**
(21) Anmeldenummer: 93110891.4
(22) Anmeldetag: 07.07.1993
(51) Int. Cl.: A61B 3/032, A61B 3/08

(54) **Sehtestgerät und zugehörige Sehtestscheibe**
Vision tester and disk providing test symbols therefore
Dispositif de test d'acuité et disque porteur de symboles s'y rapportant

(30) Priorität: 07.07.1992 DE 4222100
(43) Veröffentlichungstag der Anmeldung: 12.01.1994
(73) Patentinhaber: G. RODENSTOCK INSTRUMENTE GMBH, D-85521 Ottobrunn-Riemerling (DE)
(72) Erfinder: Wilms, Karl-Heinz, D-82275 Emmering (DE); Karl, Hans-Peter, D-81479 München (DE)
(74) Vertreter: Münich, Wilhelm, Dr.

(56) Entgegenhaltungen:
- EP-A- 0 060 986
- CH-A- 458 778
- DE-A- 4 036 964
- DE-U- 1 963 161
- US-A- 3 486 813

## Beschreibung

Die Erfindung bezieht sich auf ein Sehtestgerät zur binokularen Prüfung des Sehvermögens gemäß dem Oberbegriff des Anspruchs 1.

Ein Sehtestgerät dieser Gattung ist aus der US-A-3 486 813 bekannt. In diesem Dokument ist ein Sehtestgerät zur Prüfung des Sehvermögens beschrieben, das jeweils einen Strahlengang für das linke und das rechte Auge der untersuchten Person aufweist, den jeweils eine Blende öffnet oder schließt. Ferner ist eine drehbare Sehtestscheibe vorhanden, auf der mehrere Sätze von Sehzeichen vorgesehen sind, deren Orientierung bezüglich einer Drehung um eine zur Sehzeichenebene senkrechte Achse die zu erkennende Eigenschaft ist. Die Sehtestscheibe ist um einen Winkel von mehr als 180° drehbar, so daß jeder Satz nacheinander sowohl in den Strahlengang für das linke als auch für das rechte Auge einbringbar ist. Bei diesem bekannten Sehtestgerät sind die beiden Zeichen jedes Satzes in einer einzigen Zeile angeordnet.

Sehtestgeräte anderer Gattung sind beispielsweise aus dem DE-GM 19 63 161 oder der DE 40 36 964 A1 bekannt. Auf diese Druckschriften wird im übrigen zur Erläuterung aller hier nicht näher erläuterten Einzelheiten ausdrücklich Bezug genommen.

Die bekannten Sehtestgeräte weisen einen Strahlengang für das linke und einen für das rechte Auge der untersuchten Person auf. In jedem Strahlengang ist eine Blende vorgesehen, die den jeweiligen Strahlengang öffnet oder schließt, so daß abwechselnd eine monokulare und eine binokulare Betrachtung der in diesem Strahlengang angebotenen Sehzeichen möglich ist.

Weiterhin ist eine drehbare Sehtestscheibe vorgesehen, die gegebenenfalls wechselbar ist. Jede Sehtestscheibe ist mit mehreren Sätzen von Sehzeichen versehen. Die korrespondierenden Sätze von Sehzeichen weisen wiederum zwei Anordnungen von Sehzeichen-Zeilen auf, die durch die Drehung der Scheibe nacheinander in den Strahlengang für das linke bzw. rechte Auge einbringbar sind.

Bei den aus dem DE-GM 19 63 161 oder der DE 40 36 964 A1 bekannten Sehtestgeräten können die Sehzeichen Zeichen wie Landolt-Ringe oder E- bzw. U-Hacken sein, deren Orientierung bezüglich einer um die Sehzeichen-Ebene senkrechten Achse von der untersuchten Person wahrgenommen werden muß. Dabei weist jede Anordnung nur in einem Strahlengang vorhandene Zeilen (monokular wahrnehmbare Zeichen) und in beiden Strahlengängen an einander entsprechenden Orten vorhandene gleiche Zeilen (binokular wahrnehmbare Zeilen) auf.

Bei diesen bekannten Sehtestgeräten ist jedoch die Sehtestscheibe lediglich um 180° drehbar und somit in einen dem linken und einen dem rechten Auge zugeordneten Bereich unterteilt. Damit können auf einer Scheibe nur eine begrenzte Zahl von Sätzen von Sehzeichen, typischerweise in Abhängigkeit von der Größe der Scheibe vier bis sechs Sätze von Sehzeichen untergebracht werden. Bei Patienten mit hohem Lern-, jedoch begrenztem Sehvermögen ist es damit erforderlich, vergleichsweise häufig die Testscheibe zu wechseln, um das Sehvermögen ohne Beeinträchtigung durch Lerneffekte testen zu können. Ein Wechseln der Scheibe führt jedoch zu Störung des Untersuchungsablaufs.

Der Erfindung liegt die Aufgabe zugrunde, ein Sehtestgerät gemäß dem Oberbegriff des Patentanspruchs 1, also der aus der US-A-3 486 813 bekannten Gattung derart weiterzubilden, daß bei einer großen Sehzeichenauswahl, die das Auftreten von Lerneffekten verhindert, gleichzeitig das binokulare und monokulare Sehvermögen geprüft wird.

Eine erfindungsgemäße Lösung dieser Aufgabe ist in den Patentansprüchen 1 gekennzeichnet. Weiterbildungen der Erfindung sind Gegenstand der abhängigen Ansprüche.

Erfindungsgemäß ist das Sehtestgerät so ausgebildet, daß die Scheibe um einen Winkel von mehr als 180°, bevorzugt um 360° bzw. ein Vielfaches von 360° drehbar ist. Damit ist jede Anordnung von Zeilen eines Satzes sowohl in den Strahlengang für das linke als auch für das rechte Auge einbringbar, so daß die Zahl von Test-Sätzen gegenüber dem Stand der Technik verdoppelt wird. Da durch die erfindungsgemäß mögliche Drehung der Testscheibe um mehr als 180° jeder Satz von Sehzeichen sowohl in "aufrechter Orientierung" als auch in "gestürzter Orientierung" dargeboten wird, sind die binokular wahrnehmbaren Sehzeichen-Zeilen jedes Satzes mittig angeordnet, so daß sich zwar deren Orientierung, nicht jedoch deren Anordnung ändert.

Gemäß Anspruch 2 weist jeder Satz eine ungerade Zahl von Zeilen, bevorzugt drei Zeilen (Anspruch 3) auf, von denen eine mit dem linken Auge, die mittlere binokular und die andere mit dem rechten Auge wahrnehmbar ist.

Die Sehzeichen können im Prinzip beliebige Sehzeichen sein, sofern sie so ausgebildet sind, daß nicht die Gestalt der Sehzeichen, sondern deren Orientierung bezüglich einer Drehung um eine zur Sehzeichenebene senkrechte Achse die zu erkennende Eigenschaft ist. Insbesondere können die Sehzeichen Landolt-Ringe oder E- oder U-Hacken sein (Ansprüche 4 bis 6).

Die Erfindung wird nachstehend anhand von Ausführungsbeispielen unter Bezugnahme auf die Zeichnung exemplarisch beschrieben. Es zeigen:
- Fig. 1: den prinzipiellen Aufbau eines Sehtestgeräts,
- Fig. 2: eine Sehtestscheibe, und
- Fig. 3: einen Satz von Sehzeichen auf einer derartigen Sehtestscheibe.

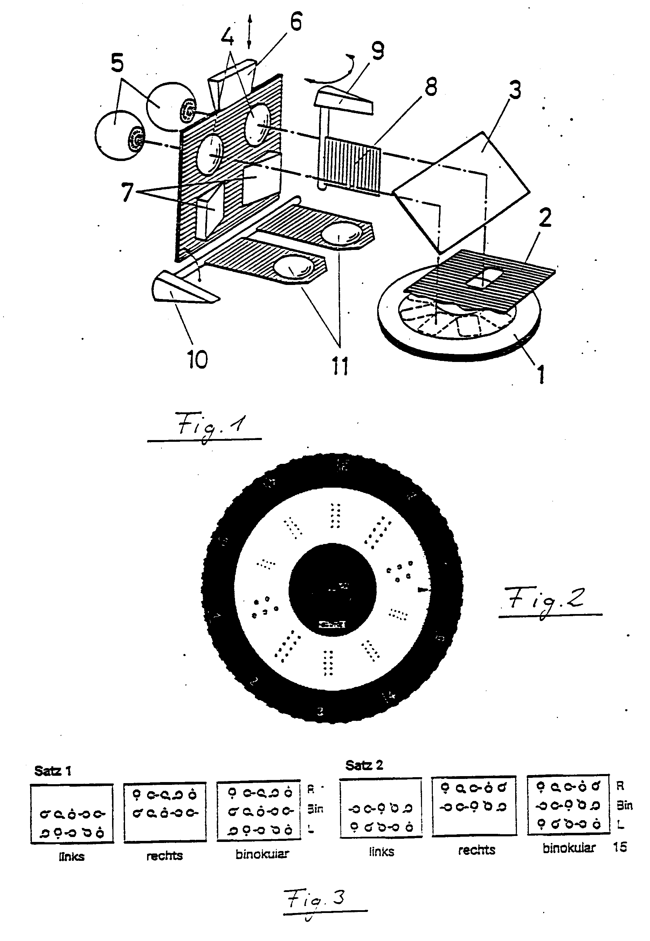

Fig. 1 zeigt den prinzipiellen Aufbau eines Sehtestgeräts. Eine auswechselbare Sehtestscheibe 1, die in Fig. 2 näher dargestellt ist, trägt bei dem gezeigten Ausführungsbeispiel fünf Paare von Sehzeichen-Sätzen, die bis auf das zu betrachtende Paar durch eine Blendenmaske 2 abgedeckt sind. Der jeweils "frei-gegebene" Sehzeichen-Satz kann von dem linken bzw. rechten Auge 5 der untersuchten Person über Linsen 4 und einen Umlenkspiegel 3 betrachtet werden. Die Linsen 4 sind in einem Schieber 6 angeordnet, der auch ein Keilpaar 7 für stereoskopische Beobachtungen trägt. Eine Schwenkblende 8 kann mittels eines Griffes 9 so gedreht werden, daß sie einen oder keinen der beiden Strahlengänge verdeckt. Alternativ kann auch ein entsprechender LCD-Verschluß vorgesehen werden. Ferner ist ein über einen Griff 10 betätigbares Vorschaltlinsenpaar 11 vorgesehen, das beispielsweise eine Umschaltung von Fernbetrachtung auf Nahbetrachtung erlaubt.

Fig. 2 zeigt eine erfindungsgemäß ausgebildete Sehtestscheibe, die um mehr als 180° drehbar ist. Dies kann beispielsweise dadurch realisiert werden, daß die bei "Norm-Scheiben" vorhandene Nase, die den Drehvorgang begrenzt, weggelassen wird. Damit kann jeder der fünf Sätze von Sehzeichen sowohl in "aufrechter Orientierung" als auch in "gestürzter Orientierung" dargeboten wird, so daß insgesamt zehn "verschiedene" Sätze von Sehzeichen dargeboten werden können.

Fig. 3 zeigt exemplarisch einen Satz von Sehzeichen mit drei Zeilen. Dabei ist mit "Satz 1" die Darbietung in "aufrechter Orientierung" und mit "Satz 2" die Darbietung des selben Satzes in "gestürzter Orientierung bezeichnet. Dabei vertauschen sich nicht nur die Orientierung der Sehzeichen - hier Landolt-Ringe - sondern auch die Anordnung der dem linken bzw. dem rechten Auge dargebotenen Zeile, nicht jedoch die Anordnung der mittig angeordneten Zeile, die binokular betrachtet werden kann.

## Patentansprüche

1. Sehtestgerät zur Prüfung des Sehvermögens, mit
- jeweils einem Strahlengang (4) für das linke und das rechte Auge (5) der untersuchten Person, den jeweils eine Blende (8) öffnet oder schließt, und
- einer drehbaren Sehtestscheibe (1), auf der mehrere Sätze von Sehzeichen vorgesehen sind, deren Orientierung bezüglich einer Drehung um eine zur Sehzeichenebene senkrechte Achse die zu erkennende Eigenschaft ist;
wobei die Sehtestscheibe um einen Winkel von mehr als 180° drehbar ist, so daß jeder Satz nacheinander sowohl in den Strahlengang für das linke als auch für das rechte Auge einbringbar ist, dadurch **gekennzeichnet**, daß die Sätze auf der Sehtestscheibe paarweise angeordnet sind, daß die Sätze eines Paares gleichzeitig in den Strahlengang für das linke bzw. das rechte Auge einbringbar sind, daß jeder Satz mehrere Sehzeichen-Zeilen aufweist,
daß die mittig angeordneten Zeilen der jeweils gleichzeitig in den Strahlengang für das linke bzw. das rechte Auge eingebrachten Sätze an einander entsprechenden Orten identisch sind, so daß sie binokular wahrnehmbar sind, und daß die außermittig angeordneten zeilen nur monokular wahrnehmbar sind.

2. Sehtestgerät nach Anspruch 1,
dadurch **gekennzeichnet**, daß jeder Satz eine ungerade Zahl von Zeilen aufweist.

3. Sehtestgerät nach Anspruch 2,
dadurch **gekennzeichnet**, daß jeder Satz drei Zeilen aufweist, von denen eine mit dem linken Auge, die mittlere binokular und die andere mit dem rechten Auge wahrnehmbar ist.

4. Sehtestgerät nach einem der Ansprüche 1 bis 3,
dadurch **gekennzeichnet**, daß die Sehzeichen Landolt-Ringe sind.

5. Sehtestgerät nach einem der Ansprüche 1 bis 3,
dadurch **gekennzeichnet**, daß die Sehzeichen E- oder U-Hacken sind.

6. Sehtestgerät nach Anspruch 4 oder 5,
dadurch **gekennzeichnet**, daß die Öffnungen der Sehzeichen in den Hauptrichtungen der Windrose angeordnet sind.

## Claims

1. Vision testing apparatus for testing visual acuity, with
- a respective beam path (4) for the left and the right eye (5) of the person being tested, and which are each closed or opened by a diaphragm (8), and
- a rotatable vision testing disc (1) upon which are provided a plurality of sets of visual symbols, whose orientation with respect to a rotation about an axis perpendicular to the plane of the visual symbol is the property to be recognised; and the vision testing disc (1) being rotatable through an angle of more than 180°, so that each set can be brought in succession into the beam path both for the right and for the left eye,
characterised in that the sets on the vision testing disc are arranged in pairs, in that the sets of a pair can be brought simultaneously into the beam path for the left or for the right eye, in that each set has a plurality of lines of visual symbols, in that the centrally-located lines of the sets respectively simultaneously brought into the beam path for the left or for the right eye are identical at points corresponding to one another, so that they may be seen in binocular vision, and in that the lines located off-centre is only visible in monocular vision.

2. Vision testing apparatus according to claim 1,
characterised in that each set has an odd number of lines.

3. Vision testing apparatus according to claims 2,
characterised in that each set has three lines, of which one may be seen by the left eye, the central one binocularly, and the other with the right eye.

4. Vision testing apparatus according to one of claims 1 to 3,
characterised in that the visual symbols are Landolt rings.

5. Vision testing apparatus according to one of claims 1 to 3,
characterised in that the visual symbols are E- or U-hooks.

6. Vision testing apparatus according to claim 4 or 5,
characterised in that the openings of the visual symbols are arranged in the principal directions of the compass rose.

## Revendications

1. Appareil de test de la vue destiné à l'examen de l'acuité visuelle, comportant
- un chemin optique (4) pour chaque oeil (5), gauche et droit, de la personne examinée, chemin qui est ouvert ou fermé par un cache (8),
- et un disque (1) de test de la vue, susceptible d'être tourné, sur lequel sont prévus plusieurs jeux de symboles visuels dont l'orientation par rapport à une rotation sur un axe perpendiculaire au plan des symboles visuels constitue la caractéristique qu'il faut reconnaître; le disque de test de la vue pouvant être tourné d'un angle supérieur à 180°, de façon telle que chaque jeu peut successivement être amené aussi bien dans le chemin optique prévu pour l'oeil gauche que dans le chemin optique prévu pour l'oeil droit, caractérisé en ce que les jeux sont disposés par paires sur le disque de test de la vue, que les jeux d'une paire peuvent être amenés simultanément dans le chemin optique prévu pour l'oeil gauche et dans le chemin optique prévu pour l'oeil droit, que chaque jeu présente plusieurs lignes de symboles optiques,
que les lignes, disposées au milieu, des jeux amenés simultanément dans le chemin optique prévu pour l'oeil gauche et dans le chemin optique prévu pour l'oeil droit, sont identiques à des emplacements qui correspondent entre eux, si bien qu'elles sont perceptibles de façon binoculaire et que les lignes disposées en dehors du milieu ne sont perceptibles que de façon monoculaire.

2. Appareil de test de la vue selon la revendication 1, caractérisé en ce que chaque jeu présente un nombre impair de lignes.

3. Appareil de test de la vue selon la revendication 2,
caractérisé en ce que chaque jeu présente trois lignes dont une peut être perçue par l'oeil gauche, celle du milieu pouvant être perçue de façon binoculaire et l'autre pouvant être perçue par l'oeil droit.

4. Appareil de test de la vue selon les revendications 1 à 3,
caractérisé en ce que les symboles visuels sont des anneaux de Landolt.

5. Appareil de test de la vue selon l'une des revendications 1 à 3,
caractérisé en ce que les symboles visuels sont des crochets en E ou en U.

6. Appareil de test de la vue selon la revendication 4 ou 5,
caractérisé en ce que les ouvertures des symboles visuels sont disposées dans les directions principales de la rose des vents.
